(19)

**Europäisches Patentamt**
**European Patent Office**
**Office européen des brevets**

(11) **EP 2 603 240 B1**

(12) **EUROPEAN PATENT SPECIFICATION**

(45) Date of publication and mention
of the grant of the patent:
**20.11.2019 Bulletin 2019/47**

(21) Application number: **11816175.1**

(22) Date of filing: **08.08.2011**

(51) Int Cl.:
*A61K 47/00* (2006.01)   *A23L 33/135* (2016.01)

(86) International application number:
**PCT/IL2011/000640**

(87) International publication number:
**WO 2012/020403 (16.02.2012 Gazette 2012/07)**

(54) **PROBIOTIC LIQUID FOOD PRODUCTS**

PROBIOTISCHE FLÜSSIGE LEBENSMITTEL

PRODUITS ALIMENTAIRES LIQUIDES PROBIOTIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.08.2010 US 371796 P**

(43) Date of publication of application:
**19.06.2013 Bulletin 2013/25**

(73) Proprietor: **Degama Smart Ltd.**
**Grand Cayman KY1-1104 (KY)**

(72) Inventor: **PENHASI, Adel**
**58671 Holon (IL)**

(74) Representative: **Maslanka Kubik, Dorota Irena**
**C/Vara de Rey 5 TER, 3, Oficina 5**
**26003 Logroño (La Rioja) (ES)**

(56) References cited:
**WO-A1-2011/004375       US-A1- 2001 021 404
US-A1- 2005 008 610      US-A1- 2005 153 018
US-A1- 2006 223 161      US-A1- 2008 193 485
US-A1- 2010 047 400      US-A1- 2010 055 083
US-A1- 2010 074 994      US-B1- 6 290 988
US-B2- 6 974 594**

- **"METHOCEL Cellulose Ethers in Aqueous
Systems for Tablet Coating", , 1 July 2002
(2002-07-01), pages 1-32, XP55044564, USA
Retrieved from the Internet:
URL:http://msdssearch.dow.com/PublishedLit
eratureDOWCOM/dh_004a/0901b8038004ab56.p
df ?filepath=/198-00755.pdf&fromPage=GetDoc
[retrieved on 2012-11-16]**

Remarks:
The file contains technical information submitted after the application was filed and not included in this specification

## Description

### Field of the Invention

The present invention relates to health food products, particularly, to liquid products containing probiotics. It is also disclosed a method of preparing a liquid product which undergoes heat treatment in at least one stage of its preparation or use, while keeping a sufficient amount of probiotic microorganisms.

### Background of the Invention

Probiotics are live microbial food supplements which beneficially affect the host by supporting naturally occurring gut flora, by competing harmful microorganisms in the gastrointestinal tract, by assisting useful metabolic processes, and by strengthening the resistance of the host organism against toxic substances. A number of organisms is used in probiotic foods, an example being bacterial genera *Lactobacillus* or *Bifidobacterium, or Lactobacillus paracasei St11 (or NCC2461), Lactobacillus fortis, Lactobacillus johnsonii La1 (= Lactobacillus LC1, Lactobacillus johnsonii NCC533) or Bifidobacterium lactis.* Probiotic organisms should survive for the lifetime of the product in order to be effective, and further they should survive the whole way through the gastrointestinal tract to the colon. Probiotic organisms are usually incorporated into milk products, such as yogurts. The need is felt to deliver the beneficial microorganisms in other foodstuff types, for example in liquid-based products especially those which undergo heat treatment in at least one stage of their preparation. The main problem in preparing liquid-based health food is the combination of high temperature and water that may destroy the whole, or a significant portion, of the included probiotics.

US 2005/0019417 A1 describes a method of preparing products containing moisture-sensitive living microorganisms including probiotics, comprising at least the steps through which a suspension of probiotics and a sugar polymer in water miscible solvent is sprayed onto a water soluble, gel-forming solid particles. By these means, the core composed of water soluble gel-forming solid particles may absorb solvent residues and provide protection to probiotics placed onto said core.

US2010/0055083 discloses a method for preparing a suspension for the encapsulation of heat and moisture sensitive substances in capsules, sachets, droplets and food compositions.

US2010/0074994 relates to a probiotic delivery system that can be consumed as a snack-food or added to a food product.

US2005/1053018 teaches of a probiotic delivery system that is preferably added to a food product and shows that compacted pellets having a volume of at least 0.02 cm$^3$, that comprise, besides viable micro-organisms, arbitrary or eligible components, such as fillers, binder, plasticizer, other functional ingredients and a coating may be added to semi-moist, moist or semi-dry products.

US6290988 teaches an encapsulated material of which at least a part of the material is kept encapsulated during heat treatment in an aqueous environment and is released during cooling after a heat treatment. The material is encapsulated in a layer of a hydrophobic film-forming material and a layer of a material having a low critical solution temperature (LCST) below the treatment temperature. The layer containing the hydrophobic material may be situated inside the layer having the LCST and have a melting point below the LCST, but it may also be situated outside the layer having the LCST and have a melting point above the LCST of said layer.

It is an object of the present invention to provide a process for preparing a nutritionally acceptable composition comprising probiotic microorganisms, the composition being resistant to high temperatures and humidity.

Another object of the present invention is to provide a process for preparing a nutritionally acceptable composition comprising probiotic microorganisms, the composition being resistant to high temperatures and humidity, said composition being added to a liquid-based food product undergoing heat treatment during its preparation.

Still another object of the invention is to provide a liquid-based product comprising viable bacteria in a sufficient amount, which liquid-based food product is supposed to undergo heat treatment during its preparation.

It is another object of the invention to provide a product comprising viable bacteria in a sufficient amount even after adding to hot water or hot aqueous-based liquid before application.

It is a further object of the invention to provide liquid-based food products containing live probiotic microorganisms during the whole process of production and/or preparation.

It is a still further object of the invention to provide liquid based food products comprising heat-stabilized probiotic composition.

It is also a further object of the invention to provide liquid-based food products containing probiotics exhibiting a long shelf life.

Other objects and advantages of present invention will appear as description proceeds.

## Summary of the Invention

It is herein disclosed a process for the preparation of heat and humidity resisting probiotic bacteria in the form of stabilized probiotic granules, for a liquid-based healthy food product, comprising the steps of i) preparing core granules containing probiotic bacteria, at least one substrate, and optionally other food grade ingredients; ii) optionally coating said core granules by at least one inner layer, thereby obtaining sealed core granules; iii) coating said optionally sealed core granules by at least one outer layer comprising a thermo-sensitive, gel forming polymer; and iv) optionally coating said core granules comprising thermo-sensitive gel with an exterior coating layer comprising at least one water soluble polymer; thereby obtaining stabilized probiotic granules for admixing to a liquid-based food product, said probiotic granules comprising heat resistant and humidity resistant probiotic bacteria. The stabilized bacteria are capable to resist higher temperature even in the humid environment during manufacturing or preparing a liquid-based food product; an example of high temperature to be resisted is a pasteurization step when manufacturing a probiotic juice, or mixing an infant powder food comprising the granules of the invention with hot water when preparing baby food.

It is herein disclosed a process, for illustrative purposes, for the preparation of a liquid-based food product comprising a heating step, the product containing active probiotic bacteria, the process comprising i) preparing stabilized probiotic granules as described above; ii) admixing said stabilized probiotic granules into a semi-final product; iii) heating the mixture of said probiotic granules/particles and said liquid-based semi-final product at a predetermined temperature and for a predetermined time period; and iv) completing said liquid based semi-final product containing said stabilized probiotic granules by cooling down said mixture, thereby obtaining said liquid-based food product containing active probiotic bacteria. The term "semi-final product" describes a stage in the preparation of a food product according to the invention, in which said food product does not yet contain all the components or has not yet passed all the preparation steps, being not yet ready for the consumption. It is herein also disclosed for illustrative purposes, the process for the preparation of a liquid-based food product comprising a heating step, comprises i) preparing stabilized probiotic granules as described above; ii) admixing said stabilized probiotic granules/particles into a semi-final product comprising an infant powder food product, thereby obtaining a probiotic infant powder food product containing stabilized probiotic granules/particles; and iii) shortly before intended consumption of said probiotic infant powder food product, adding to said product cold water and heating or alternatively adding hot water, while keeping the mixture at a predetermined temperature for a predetermined time period. Said outer layer, composed of a thermo-sensitive gel forming polymer, forms a solid gel surrounding the probiotics core during said heating step, thereby preventing the transmission of the heat and humidity to the probiotics, while said gel dissolves after said cooling, allowing the pro-biotic material to be released in a desired liquid-based product.

The invention is defined by the claims.

The invention provides stabilized probiotic granules for admixing to a liquid-based food product, resistant to heating in an aqueous environment, comprising thermo-reversible gel-forming polymer. The stabilized probiotic granules of the invention comprise a core of probiotic bacteria in a substrate or mixed with a substrate, at least one inner layer coating said core, and at least one outer layer comprising a thermo-reversible gel forming polymer. The granules comprise a core of probiotic bacteria with a substrate, at least one inner layer coating said core, and at least one outer layer comprising a thermo-reversible gel forming polymer, and optionally at least one exterior layer comprising a water soluble polymer or erodible polymer. Other granules, which do not form part of the invention, comprise a core of probiotic bacteria in a substrate, at least one outer layer comprising a thermo-reversible gel- forming polymer, and at least one exterior layer comprising a water soluble polymer or erodible polymer. Said substrate may comprise a component selected from the group consisting of supplement for bacteria, stabilizer, filler, binder, and a mixture thereof. Said substrate may comprises a prebiotic saccharide, wherein said inner layer may comprise a water soluble or erodible polymer, and wherein said outer layer may comprise a thermo-sensitive sol-gel forming polymer. In one embodiment, said substrate comprises a prebiotic saccharide, wherein said inner layer comprises a water soluble or erodible polymer, said outer layer comprises a thermo-sensitive sol-gel forming polymer, and wherein said exterior layer comprises a water soluble polymer or erodible polymer. Said substrate preferably comprises a prebiotic saccharide, wherein said outer layer comprises a thermo-sensitive sol-gel forming polymer, and wherein said exterior layer comprises a water soluble polymer or erodible polymer. The granules of the invention preferably have an outer layer composed of a thermo-sensitive gel-forming polymer which forms a solid gel surrounding the core granules when heated, thereby preventing the transmission of the heat and humidity to the probiotic bacteria, while said gel dissolves after cooling, allowing said bacteria to be released in a liquid-based product.

When using the term "liquid-based food product", intended is a product which has a high content of water, or which is intended for dispersing in water. Thus a liquid-based food product according to the invention may be a product having the form of liquid, suspension, emulsion, or paste, but it may be a powder intended for dispersing in water or water-based liquid, such as milk. In a preferred embodiment, said granules comprise a core of probiotic bacteria in a substrate, at least one inner layer coating said core, at least one outer layer comprising a thermo-reversible gel-forming polymer, and optionally at least one outermost layer comprising a water soluble polymer. Said substrate may comprise a component selected from the group consisting of supplement for bacteria, stabilizer, buffering agent, chelating agent, filler, binder, and a mixture thereof. Said granules comprise, in one embodiment of the invention, a prebiotic saccharide in the core, a water soluble or erodible polymer in said inner layer, importantly a thermo-sensitive, sol-gel forming polymer in the outer layer, and a water soluble or erodible polymer in said outermost layer.

It is herein disclosed a food product, for illustrative purposes, selected from infant food products, infant food powder compound, yogurt, dairy products, nectars, fruit juices, and energetic drinks/beverages, which product is a health food product comprising probiotic bacteria which were heat-stabilized as described above. According to the invention, the healthy food product is an infant powdered food comprising the granules according to the invention.

Thus, it is herein disclosed heat-processed or heat-processible healthy food beneficially affecting the consumer's intestinal microbial balance, wherein said heat-resistance and heat-processability are ensured by coating probiotic cores by layers which limit the transmission of heat and humidity to the probiotic bacteria and so increase their resistance during a heat step-comprising process.

Particularly, the object of the invention are stabilized probiotic granules for admixing to a liquid-based food product, resistant to heating in an aqueous environment, comprising

i) a core of probiotic bacteria in a substrate or mixed with a substrate;

ii) at least one first layer adjacent to the core which is an outer layer comprising a thermo-reversible gel-forming polymer having a lower critical solution temperature (LCST) of at least 45 ○C selected from HPC LF or HPC EF in combination with Poloxamer 124,

iii) an inner layer, which is layered between said core of probiotic bacteria and said first layer (outer layer) comprising the thermo-reversible gel forming polymer, wherein said inner layer is selected from the group consisting of water soluble or erodible polymers, wherein said inner layer comprises HPMC E5 and polyethylene glycol;

iv) optionally a second layer adjacent to said outer layer, which is an exterior layer comprising a water soluble or erodible polymer for enhancing the dissolution of said thermo-reversible gel forming polymer on cooling below its lower critical solution temperature (LCST), wherein said second layer comprises HPMC E5 and polyethylene glycol.

The object of the invention is also an infant powdered food comprising said stabilized granules.

**Brief Description of the Drawing**

The above and other characteristics and advantages of the invention will be more readily apparent through the following examples, and with reference to the appended drawing, wherein:

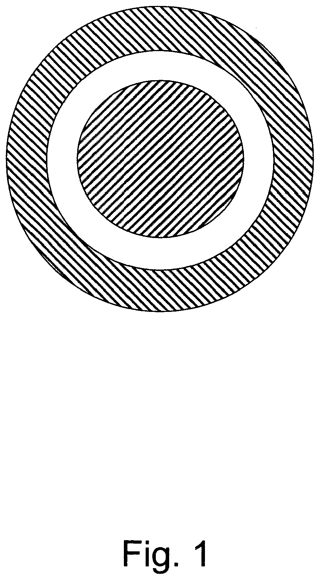

Fig. 1.   shows a schema of a multiple-layered capsule according to one embodiment of the invention, to be comprised in healthy food; the encapsulation is designed to provide probiotic bacteria with maximum heat resistance during the heating step of either manufacturing process or preparation process; the core comprises probiotic bacteria and an absorbing substrate; the first layer adjacent to the core is the inner first sealing layer; the outer layer adjacent to said inner layer is the outer, thermo-reversible gel forming layer; alternatively, the core comprises probiotic bacteria and an absorbing substrate; the first layer adjacent to the core is the outer, thermo-reversible gel forming layer; the second layer adjacent to said outer layer is the exterior layer;

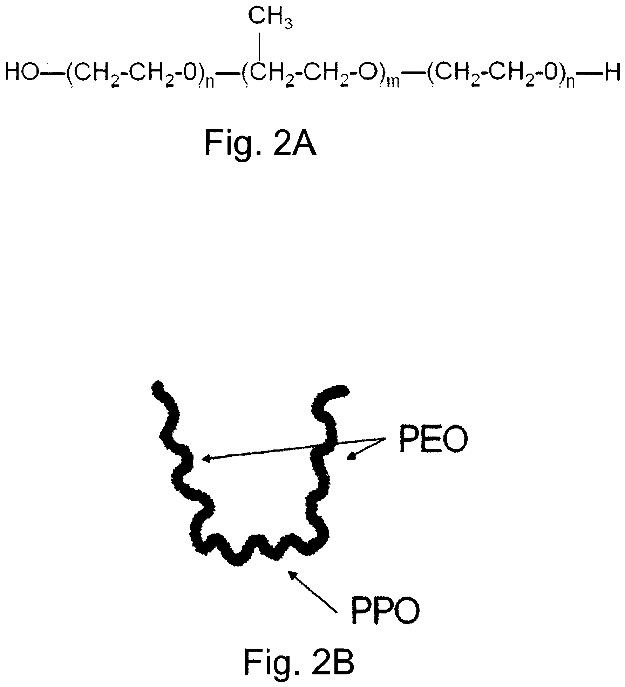

Fig. 2.   shows the structure of Pluronic, comprising an ABA tri-block copolymer comprising polypropylene oxide and polyethylene oxide; Fig. 2A shows the molecular structure; and Fig. 2B is a schematic representation of the three-block polymeric chain;

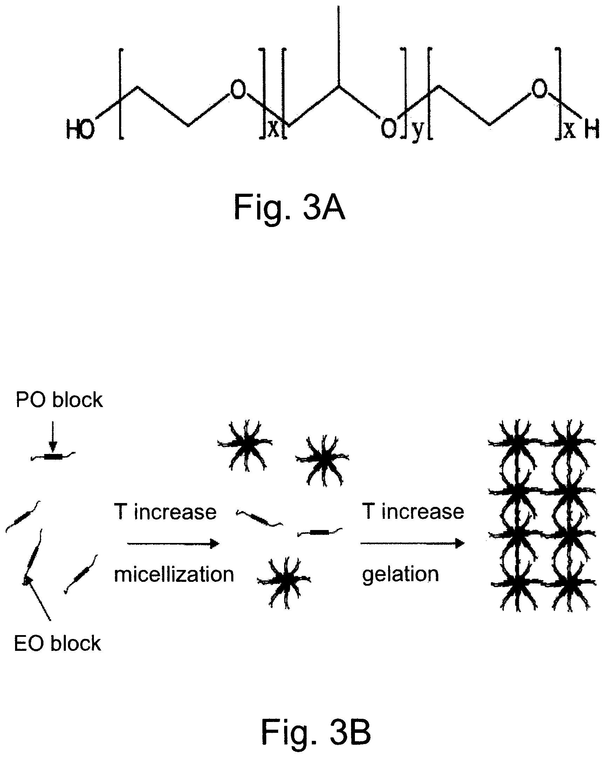

Fig. 3.   shows the sol-gel transition of Pluronic, an ABA triblock copolymer of polypropylene oxide and polyethylene oxide, as a function of temperature; the presence of polymer blocks having certain cloud point imparts the polymer with the property of being converted into a hydrophobic state at a temperature higher than the cloud point, and of being converted into a hydrophilic state at a temperature lower than the cloud point temperature; this results from the thermodynamic property of hydrophobic bonds increasing in strength with increasing temperature (and conversely decreasing in strength with decreasing temperature); Fig. 3A shows the molecular structure; and Fig. 3B is a schematic representation of the gelation process;

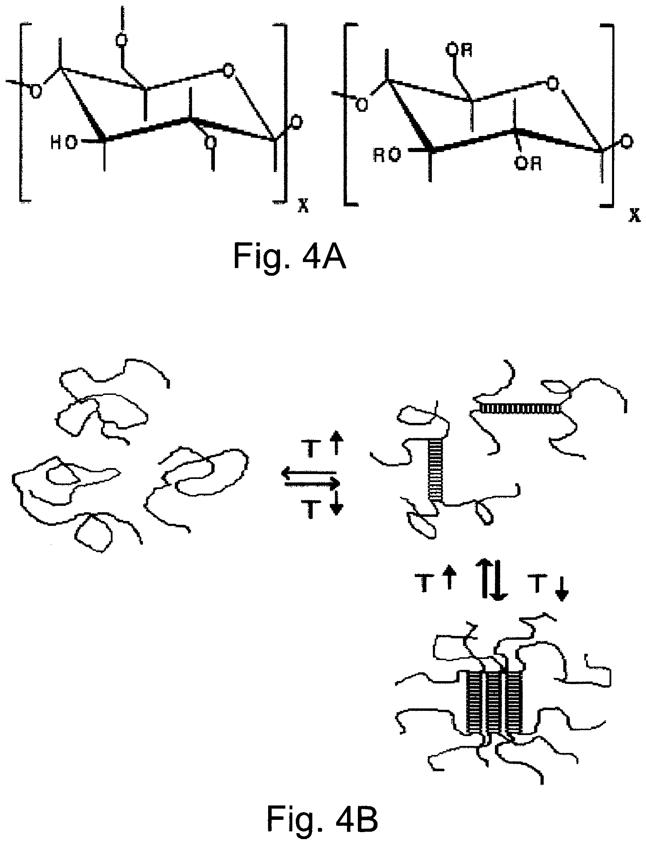

Fig. 4.   shows the sol-gel transition of a cellulose derivative such as hydroxyl propyl cellulose (HPC) as a function of temperature; an increase over a critical temperature results in chain-chain interactions, including hydrophobic effects and hydrogen bonding, to dominate over chain-water hydrogen bonding; on the other hand, upon decreasing temperature below a critical temperature, water hydrogen bonding dominates over chain-chain interactions enabling the dissolution of the polymer; Fig. 4A shows the molecular structure; and Fig. 4B is a schematic representation of the molecular interactions;

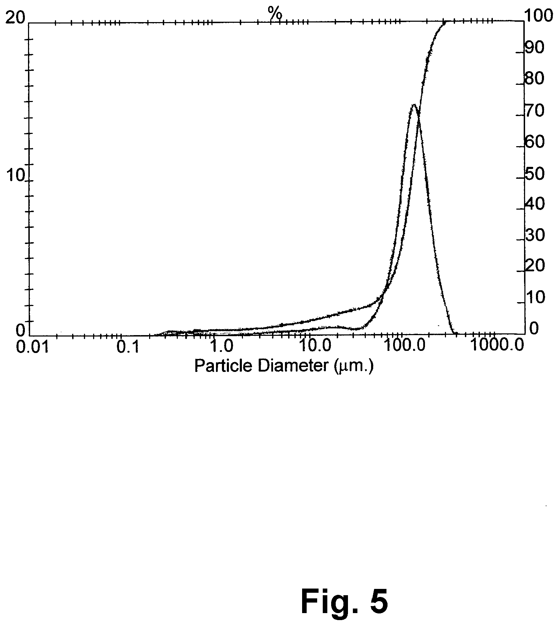

Fig. 5.   is a graph of particle size distribution of microencapsulated *Bifidobacterium lactis* (BL818), made according to an embodiment of the invention described in Example 1, in water after heating at 70°C and cooling down;

hydroxypropyl cellulose (HPC LF) was used as thermo-sensitive sol-gel film coating with a weight gain of 70%;

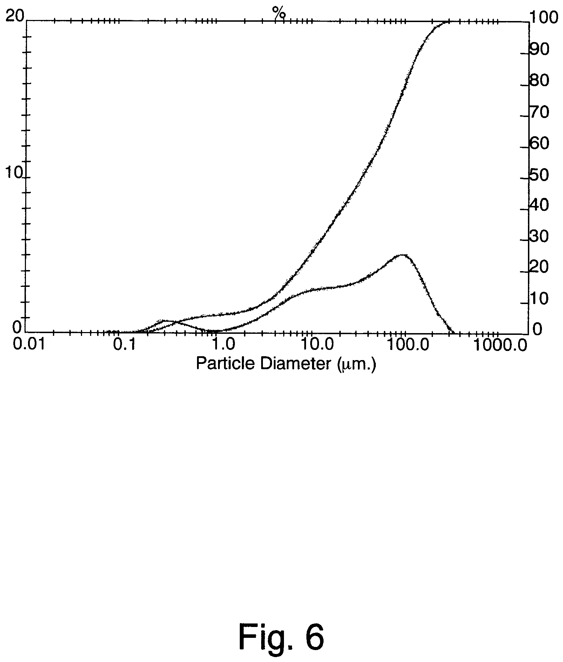

Fig. 6.  is a graph of particle size distribution of microencapsulated *Bifidobacterium lactis* (BL818), in the form of stabilized granules according to the invention, in water after heating at 70°C and cooling down; hydroxypropyl cellulose (HPC LF) was used as thermo-sensitive sol-gel film coating with a weight gain of 50%; and

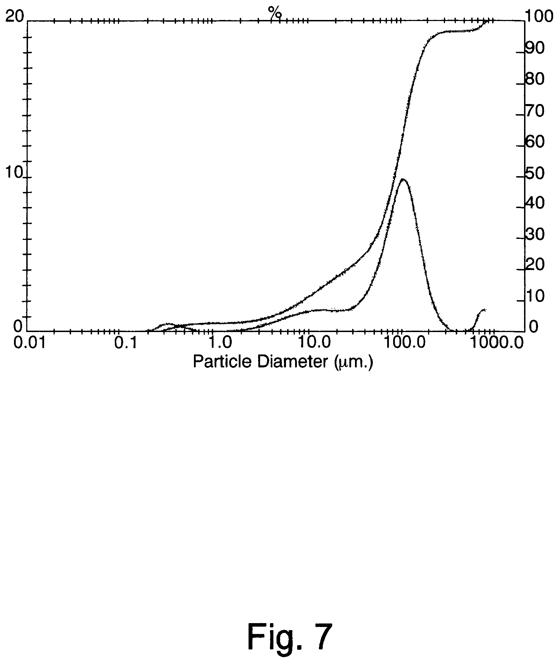

Fig. 7.  is a graph of particle size distribution of microencapsulated *Bifidobacterium lactis* (BL818), made according to an embodiment of the invention described in Example 2, in water after heating at 70°C and cooling down; hydroxypropyl cellulose (HPC EF) was used as thermo-sensitive sol-gel film coating with a weight gain of 70%

## Detailed Description of Preferred Embodiments

It has now been found that probiotic bacteria may be surprisingly efficiently stabilized for use in a heat-step comprising process by coating with a sol-gel forming polymer. The bacteria were formulated in a granulated core coated with one or more coating layer, thereby obtaining probiotic compositions providing viable probiotic organisms even after heating at relatively high temperatures at high humidity, the composition being further stable on storage and capable of administering viable bacteria to the gastrointestinal tracts after the oral administration. The invention provides granular probiotics to be used as healthy food additives. It is herein also disclosed for illustrative purposes a process for the preparation of liquid-based food, such as infant food powder compound which is substantially suspended in hot water (about 70 ° C), fruit juices, nectars, yogurts, milk-based dairy products and energetic drinks containing heat resisting probiotics.

It is herein disclosed a process for the preparation of liquid-based food, comprising the steps of i) preparing a core (granules) that comprises probiotic bacteria; ii) optionally coating said core (granules) by at least one inner layer comprising a water soluble polymer for preventing humidity penetration into the core (granules); iii) coating said granules by at least one outer layer comprising a thermo-reversible gel-forming (sol-gel) polymer for resisting heat and humidity, thereby obtaining a stabilized probiotic granule; iv) optionally coating said core (granules) by at least one outermost layer comprising a water soluble polymer; v) admixing said stabilized probiotic granules to a liquid-based food pre-product (semi-final product); and vi) completing the preparation the said liquid-based food pre-product containing said stabilized probiotic granules by heat treatment at predetermined temperature for predetermined time. As disclosed herein for illustrative purposes, said stabilized probiotic granules are added to a solid-based food product such as powder product (like infant food powder compound), which should be eventually added to hot water (up to 70°C) before using, and allowed to cool down before consumption. In an important embodiment of the invention, said stabilized probiotic granule has a core comprising probiotic bacteria and a substrate, to which said bacteria are absorbed or with which they are granulated, said core containing additionally other nutritionally acceptable excipients; the granule has optionally further an inner layer of water soluble polymer; the granule has an outer layer of thermo-sensitive (thermo-reversible) gel forming polymer having a sol-gel transition (transition temperature); the granule has optionally an exterior layer of water soluble polymer. In another important embodiment of the invention, both said inner layer and said outer layer comprise thermo-sensitive gel forming polymers having a sol-gel transition, but with different molecular weights or viscosities. In another important embodiment of the invention, both said inner layer and said exterior layer comprise similar polymers having similar molecular weights or viscosities or similar polymers but with different molecular weights or viscosities. In another important embodiment of the invention, said stabilized probiotic granule has a core comprising probiotic bacteria and a substrate in which said bacteria are granulated or absorbed, said granule containing additional excipients, and further a single layer of thermo-sensitive gel forming polymer having a sol-gel transition. Said stabilized probiotic granule, which in this case does not form part of the invention, has a core comprising probiotic bacteria and a substrate in which said bacteria are absorbed or granulated and said granule containing additionally other acceptable excipients; an outer layer of thermo-sensitive gel forming polymer having a sol-gel transition; an exterior layer of water soluble polymer. In another embodiment of the invention, said stabilized probiotic granule has a core comprising probiotic bacteria and a substrate in which said bacteria are absorbed or granulated and said granule containing additionally other acceptable excipients; an inner layer of water soluble polymer; and two outer layers including a lower, enteric layer providing gastric resistance, and an upper layer of thermo-sensitive gel forming polymer having a sol-gel transition.

For illustrative purposes, the process as disclosed herein comprises granulating probiotic bacteria, coating them by at least one inner layer for resisting humidity, at least one outer layer for resisting production (manufacturing) heat and humidity, wherein said resisting occurs at a predetermined production temperature for predetermined heat process time, after which said second layer is swelled forming gel during exposing to high temperature, so preventing the penetration of the hot liquid into the core containing said probiotics, allowing the probiotic bacteria to be safe from heating, and then to be released into a liquid food product, when the outer layer or exterior layer dissolves on cooling. A process includes, in a preferred disclosure, preparing a stabilized probiotic granule having i) a core with probiotic bacteria which may contain at least one stabilizing agent, antioxidant, sugar, filler, binder, and other excipients, and further having ii) an inner layer coating the core comprising a water soluble polymer preventing the permeation of water and humidity into the core, and further having also iii) an outer layer coating said core and said inner layer, where said outer layer comprises at least one thermo-reversible gel forming polymer having a sol-gel transition temperature. In another disclosure, the preferred process comprises granulating probiotic bacteria, coating them by at least one outer layer (first layer) for resisting production (manufacturing) heat and humidity, wherein said resisting occurs at a predetermined production temperature for predetermined heat process time, after which said outer layer is swelled forming gel during exposing to high temperature, so preventing the penetration of the hot liquid into the core containing said probiotics, allowing the probiotic bacteria to be safe from heating, and then to be released into said liquid food product, when the outer layer dissolves on cooling; and at least one outermost layer (second layer) for enhancing the dissolution of said outer layer (first layer) on cooling. In another preferred disclosure, the preferred process comprises granulating probiotic bacteria, coating them by at least one inner layer for resisting humidity (first layer); at least one outer layer (second layer) for resisting production (manufacturing) heat and humidity, wherein said resisting occurs at a predetermined production temperature for predetermined heat process time, after which said outer layer is swelled forming gel during exposing to high temperature, so preventing the penetration of the hot liquid into the core containing said probiotics, allowing the probiotic bacteria to be safe from heating, and then to be released into said liquid food product, when the outer layer dissolves on cooling; and at least one exterior layer (third layer) for enhancing the dissolution of said outer layer (first layer) on cooling.

A process as disclosed herein includes, in a preferred disclosure, preparing a stabilized probiotic granule having i) a core with probiotic bacteria and which may contain at least one stabilizing agent, antioxidant, sugar, filler, binder, and other excipients and further having ii) an inner layer coating the core comprising of a water soluble polymer preventing the permeation of water and humidity into the core, and further having also iii) an outer layer coating said core and said inner layer, where said outer layer comprises at least one thermo-reversible gel forming polymer having a sol-gel transition temperature, wherein said inner layer comprises at least one thermo-reversible gel forming polymer having a sol-gel transition temperature which can chemically be either similar to or different from said outer layer.

The invention provides a stabilized probiotic granule comprising i) a core comprising probiotic bacteria and a substrate on which said bacteria are absorbed or coated; ii) an inner layer comprising a polymer preventing the permeation of water and humidity into the core coating said core; iii) at least one outer layer, coating said core and said inner layer, comprising thermo-sensitive polymer having a sol-gel transition temperature; and iv) optionally an exterior layer comprising a polymer enhancing the dissolution of said outer layer (first layer) on cooling. Said core preferably further comprises one or more supplemental agents for said bacteria, for example prebiotic oligosaccharides.

The invention provides stabilized probiotic granules for admixing to a liquid-based food product, resistant to heating in an aqueous environment, comprising:

i) a core of probiotic bacteria in a substrate or mixed with a substrate;
ii) at least one first layer adjacent to the core which is an outer layer comprising a thermo-reversible gel-forming polymer having a lower critical solution temperature (LCST) of at least 45 °C selected from HPC LF or HPC EF in combination with Poloxamer 124,
iii) an inner layer, which is layered between said core of probiotic bacteria and said first layer (outer layer) comprising the thermo-reversible gel forming polymer, wherein said inner layer is selected from the group consisting of water soluble or erodible polymers, wherein said inner layer comprises HPMC E5 and polyethylene glycol;
iv) optionally a second layer adjacent to said outer layer, which is an exterior layer comprising a water soluble or erodible polymer for enhancing the dissolution of said thermo-reversible gel forming polymer on cooling below its lower critical solution temperature (LCST), wherein said second layer comprises HPMC E5 and polyethylene glycol.

In a preferred embodiment of the invention, said probiotic bacteria comprise a genus selected from *Lactobacillus* and *Bifidobacterium*. The stabilized probiotic core granule or core mixing according to the invention is a coated granule, comprising at least two layered phases, for example a core and two coats, or a core and three or more coats. Usually, one of the coats contributes mainly to prevention of water or humidity penetration into the core during the coating of the outer layer or during later stages, such as when the ultimate multilayered probiotics are suspended in a liquid-based product during the preparation of said liquid-based product or during the coating processes. Another outer coat contributes to the heat resistance during the liquid-based food product processing. Another exterior coat contributes to the enhancing dissolution of said outer thermo-sensitive gel forming layer on cooling. Usually, it is one of the layers that contributes maximally to said heat resistance and water or humidity penetration into the core; however, the stabilized probiotic granule of the invention may comprise more layers that contribute to the process stability of the bacteria, as well as to their stability during storing said food and during safe delivery of the bacteria to the intestines. Likewise, the two inner and exterior coats may be the same polymers with either same or different viscosities or molecular weights. Likewise, one thermo-sensitive gel-forming polymer may be used for coating the core particles, whereby one single coating layer provides protection against water and humidity penetration into the core, as well as resistance against heat and humidity.

It is herein also disclosed a process, for illustrative purposes, of manufacturing healthy food, comprising i) mixing a suspension of probiotic bacteria with a substrate and with supplemental agents for the bacteria, thereby obtaining a core mixture; ii) coating particles of said core mixture with an inner water soluble polymer; iii) coating said coated particles with an outer polymer layer, which said outer polymer layer confers stability to said bacteria under the conditions of heat and humidity, thereby obtaining particles coated with two layers. It is also disclosed a process of manufacturing healthy food, comprising i) mixing a suspension of probiotic bacteria with a substrate and with supplemental agents for the bacteria, thereby obtaining a core mixture; ii) optionally coating particles of said core mixture with an inner water soluble polymer; iii) coating said coated particles with an outer polymer layer; optionally coating said coated particles of said core mixture with an exterior water soluble polymer, which said outer polymer layer confers stability to said bacteria under the conditions of heat and humidity, thereby obtaining particles coated with three layers. It is also disclosed a process of manufacturing healthy food, comprising i) granulation of probiotic bacteria with substrates and with supplemental agents for the bacteria, thereby obtaining core granule particles; ii) coating particles of said core granule with an inner water soluble polymer; iii) coating said coated particles with an outer polymer layer, which said outer polymer layer confers stability to said bacteria under the conditions of heat and humidity, thereby obtaining particles coated with two layers. It is also disclosed a process of manufacturing healthy food, comprising i) granulation of probiotic bacteria with substrates and with supplemental agents for the bacteria, thereby obtaining core granule particles; ii) coating particles of said core granule with an outer polymer layer, which said outer polymer layer confers stability to said bacteria under the conditions of heat and humidity; iii) coating said coated particles with an exterior water soluble polymer, thereby obtaining particles coated with two layers. It is also disclosed a process of manufacturing healthy food, comprising i) granulation of probiotic bacteria with substrates and with supplemental agents for the bacteria, thereby obtaining core granule particles; ii) coating particles of said core granule with an inner water soluble polymer; iii) coating said coated particles with an outer polymer layer, which said outer polymer layer confers stability to said bacteria under the conditions of heat and humidity; iv) coating said coated particles with an exterior water soluble polymer, thereby obtaining particles coated with three layers.

In a preferred process of manufacturing probiotic food, an aqueous suspension of probiotic bacteria is mixed with at least one substrate and at least one oligosaccharide, and optionally other food grade additives such as stabilizers, fillers, binders, antioxidant, and etc., thereby obtaining a wet core mixture; particles of said wet core mixture are dried, thereby obtaining a core mixture; particles of said core mixture are coated with an inner coating layer polymer preventing or reducing the penetration of water or humidity into said core, thereby obtaining water sealed coated particles; said water sealed coated particles are coated with a thermo-reversible gel-forming polymer. Said at least one substrate may comprise galactan, galactose or a mixture thereof, said at least one oligosaccharide may comprise, galactan, maltodextrin, and trehalose, said other food grade additives comprise stabilizer, antioxidant, filler and binder, said inner coating layer polymer may comprise hydroxypropyl methyl cellulose, and/or polyvinyl-based polymer, and said thermo-reversible gel forming polymer may comprise hydroxypropyl cellulose and/or copolymer of polypropylene glycol and polyethylene glycol (Pluronic). In another preferred process of manufacturing probiotic food, an aqueous suspension of probiotic bacteria is mixed with at least one substrate and at least one oligosaccharide, and optionally other food grade additives such as stabilizers, fillers, binders, antioxidant, and etc., thereby obtaining a wet core mixture; particles of said wet core mixture are dried, thereby obtaining a core mixture; particles of said core mixture are coated with an outer coating layer comprising thermo-reversible gel forming polymer, thereby obtaining a thermo-sensitive polymer coated core mixture; particles of said thermo-sensitive polymer coated core mixture are coated with an exterior water soluble polymer enhancing the dissolution of said thermo-reversible gel forming polymer on cooling. Said at least one substrate may comprise galactan, galactose or a mixture thereof, said at least one oligosaccharide may comprise, galactan, maltodextrin, and trehalose, said other food grade additives comprise stabilizer, antioxidant, filler and binder, said thermo-reversible gel forming polymer may comprise hydroxypropyl cellulose and/or copolymer of polypropylene glycol and polyethylene glycol (Pluronic); and said outermost coating layer polymer may comprise hydroxypropyl methyl cellulose, and/or polyvinyl-based polymer.

Another preferred process of manufacturing micro encapsulated probiotic bacteria as disclosed herein includes the following steps:

1. Drying mix of probiotics mixture, with at least one substrate and at least one oligosaccharide, and optionally other food grade additives such as stabilizers, fillers, binders, antioxidant, and etc., thereby obtaining a core mixture.
2. Granulating said core mixture using a binder solution in purified water, thereby obtaining a core granule.
3. Optionally coating particles of said core granule with an inner coating layer polymer preventing or reducing the penetration of water or humidity into said core, thereby obtaining water sealed coated particles.
4. Coating said water-sealed coated particles with a thermo-reversible gel-forming polymer.
5. Optionally coating particles of said core granule with an exterior coating layer polymer enhancing the dissolution of said thermo-reversible gel forming polymer on cooling below its cloud point or its lower critical solution temperature (LCST).

The invention provides probiotic compositions comprising the stabilized probiotic granules described above, which granules exhibit high heat resistance and long storage stability. The composition as disclosed for illustrative purposes is a healthy food product, for example food product selected from the group consisting of infant food products, infant food powder compounds, yogurts, dairy products, nectars, and fruit juices. According to the invention, the healthy food product is an infant powdered food comprising the granules according to the invention. Said food product was exposed to higher than ambient temperature during either production process or preparation process.

It is herein also disclosed for illustrative purposes a process for the preparation of liquid-based food products containing probiotics, such as probiotic fruit juices, nectars, yogurts, milk-based dairy products, energetic drinks/beverages, and infant food powder compound to be suspended in hot water (about 70°C). A mixture that comprises probiotic material is prepared and then converted to granules, e.g., by fluidized bed technology such as Glatt or turbo jet, Glatt or an Innojet coater/granulator, or a Huttlin coater/granulator, or a Granulex. The resulting granules, are encapsulated by a first layer, preferably a water soluble polymer layer for resisting water or humidity penetration into the core granule which may occur in the further steps of heat resistance probiotic composition preparation then by a second layer with a thermo-sensitive gel forming polymer for resisting heat at a predetermined temperature for a predetermined time period. Alternatively, the resulting granules, are encapsulated by an outer layer (first layer) with a thermo-sensitive gel forming polymer for resisting heat at a predetermined temperature for a predetermined time period then by a second layer (exterior layer) preferably a water soluble polymer layer for enhancing the dissolution of said thermo-sensitive gel forming polymer on cooling below its cloud point or its lower critical solution temperature (LCST). Alternatively, the resulting granules, are encapsulated by a first layer (inner layer), preferably a water soluble polymer layer for resisting water or humidity penetration into the core granule which may occur in the further steps of heat resistance probiotic composition preparation then by a second layer (outer layer) with a thermo-sensitive gel-forming polymer for resisting heat at a predetermined temperature for a predetermined time period then by a third layer (exterior layer) preferably a water soluble polymer layer for enhancing the dissolution of said thermo-sensitive gel forming polymer on cooling below its cloud point or its lower critical solution temperature (LCST). Then resulting micro-encapsulated probiotics according to the above steps is introduced to a liquid-based product which must undergo a heating step during its preparation process. Alternatively the above resulting microencapsulated probiotics can be added to a food product being a solid powder mixture, such as an infant food powder, which should further be added to a hot water (usually up to 70°C). During the exposure of the above resulted microencapsulated probiotics to heat, during the preparation process of liquid-based food product, the outer layer, which is composed of a thermo-sensitive gel forming polymer, forms a solid gel surrounding the probiotics core granule preventing the transmission of the heat and humidity to the probiotics. After lowering the temperature, the outer thermo-sensitive gel forming layer dissolves, allowing the pro-biotic material to be released in the liquid-based product. The double or triple encapsulated granules can advantageously be added to a solid powder mixture food product such as an infant food powder compound. In this case before consuming the solid powder, it should be added to a hot water which has up to 80°C preferably 70°C, to prepare an appropriate suspension. Again, during the exposure of the microencapsulated probiotics, according to the present invention, to the hot water, as described above, the most outer layer which is composed of a thermo-sensitive gel forming polymer forms a solid gel surrounding the probiotics core, preventing the transmission of the heat to the probiotics. After letting the suspension cool down, the outer thermo-sensitive gel forming layer is dissolved to allow the pro-biotic material to be released in the infant suspension. The invention thus provides a liquid-based food product containing probiotics which survive the heating step needed during the preparation of the product for human uses, such as, yogurt, dairy products, nectars, and fruit juice. The product consists of: a) encapsulated granules, made of a mixture that comprises probiotic material which is dried and converted to core granules to be encapsulated by optionally an inner layer (first layer), preferably a water soluble polymer layer for resisting water and humidity penetration into the core granules, and by an outer layer (second layer) comprises at least one thermo-sensitive gel forming polymer resisting transition heat and humidity in the core granules for a predetermined manufacturing temperature and time, after which the second layer is being dissolved upon cooling down to allow the pro-biotic material to be released in the liquid-based food product, and optionally by an exterior layer (third layer layer), preferably a water soluble polymer layer for enhancing the dissolution of said thermo-sensitive gel forming polymer on cooling below its cloud point or its lower critical solution temperature (LCST); and b) an infant food product or an infant food powder compound to which the micro-encapsulated granules according to the present invention are previously added. Before consumption, the mixture of infant food product or infant food powder compound and the micro-encapsulated granules according to the present invention is added into a hot water (preferably about 70° C).

So, described is a process for preparing probiotic bacteria capable of heating during manufacturing or preparing food with high rates of survivability. The first step in making said probiotic food is preparing a core or granules comprising dried probiotic bacteria, These granules are then encapsulated by optionally a first water soluble polymer layer. The first layer helps to resist the water and humidity penetration into the granules. The second layer is then created comprising at least one thermo-sensitive gel forming polymer. Optionally a third layer is then created comprising at least one a water soluble polymer layer for enhancing the dissolution of said thermo-sensitive gel-forming polymer on cooling below its cloud point or its lower critical solution temperature (LCST).The encapsulated granules are then added to a liquid based food product right before the final preparation. The second layer is dissolved after cooling the liquid-based food product at the end of the preparation process, allowing the probiotic material to be released from the encapsulated granules into the liquid-based product.

The inner coating layer: According to further features of the preferred embodiments of the invention, the encapsulated probiotics further comprise an inner coating, which is layered between the inner core and the thermo-reversible outer sol-gel coating layer. Example of materials that may be used for the first coating layer is selected from the group consisting of water soluble or erodible polymers such as, for example, Povidone (PVP: polyvinyl pyrrolidone), Copovidone (copolymer of vinyl pyrrolidone and vinyl acetate), polyvinyl alcohol, Kollicoat Protect (BASF) which is a mixture of Kollicoat IR (a polyvinyl alcohol (PVA)-polyethylene glycol (PEG) graft copolymer) and polyvinyl alcohol (PVA), Opadry AMB (Colorcon) which is a mixture based on PVA, Aquarius MG which is a cellulose-based polymer containing natural wax, lecithin, xanthan gum and talc, low molecular weight HPC (hydroxypropyl cellulose), low molecular weight HPMC (hydroxypropyl methylcellulose) such as hydroxypropylcellulose (HPMC E3 or E5) (Colorcon), methyl cellulose (MC), low molecular weight carboxy methyl cellulose (CMC), low molecular weight carboxy methyl ethyl cellulose (CMEC), low molecular weight hydroxyethylcellulose (HEC), low molecular weight hydroxyl ethyl methyl cellulose (HEMC), low molecular weight hydroxymethylcellulose (HMC), low molecular weight hydroxymethyl hydroxyethylcellulose (HMHEC), low viscosity of ethyl cellulose, low molecular weight methyl ethyl cellulose (MEC), gelatin, hydrolyzed gelatin, polyethylene oxide, water soluble gums, water soluble polysaccharides, acacia, dextrin, starch, modified cellulose, water soluble polyacrylates, polyacrylic acid, polyhydroxyethylmethacrylate (PHEMA),polymethacrylates, their copolymers, and/or mixtures thereof.

More preferably the inner first coating layer polymers are low molecular weight HPMC (hydroxypropyl methylcellulose) such as hydroxypropylcellulose (HPMC E3 or E5) (Colorcon), polyvinyl alcohol, Kollicoat Protect (BASF) which is a mixture of Kollicoat IR (a polyvinyl alcohol (PVA)-polyethylene glycol (PEG) graft copolymer) and polyvinyl alcohol (PVA) and silicon dioxide, Opadry AMB (Colorcon) which is a mixture based on PVA, and Aquarius MG which is a cellulose-based polymer containing natural wax. Theses polymers provide superior barrier properties against water/humidity penetration into the core. Optionally the inner first coating layer may further comprise an excipient which may be at least one of a glidant, a surfactant, filler, a solubilizer, and a buffering agent.

According to the invention, the inner layer comprises HPMC E5 and polyethylene glycol.

Outer heat resisting coating layer: The outer coating layer provides heat resistance and also prevents the water and humidity penetration into the core. This coating layer comprises a thermo-reversible (thermo-sensitive) sol-gel forming polymer. Thermo-reversible sol-gel forming polymer or thermo-sensitive sol-gel forming polymer belongs to a category of physical transitions which do not require use of organic solvents, chemical cross-linking reactions or externally operated devices (e.g. photopolymerization) in order to form gel upon contact with aqueous solution at a predetermined situation, and thus are less likely to induce toxicities to the surrounding media. Temperature sensitive polymers show abrupt changes in their solubility as a function of environmental temperature. This property was employed to develop aqueous solutions of these polymers which undergo sol-gel transition in response to temperature changes. At lower critical solution temperature (LCST), the interaction forces (hydrogen bonding) between water molecules and polymer become unfavorable compared to polymer-polymer and water-water interaction and phase separation occurs as the polymer dehydrates. Consequently, aqueous polymer solutions display low viscosity at ambient temperature but exhibit a sharp increase in viscosity following temperature rise, forming a semi-solid gel. One major advantage of formulations based on such polymers is their ability to form a stable gel which does not dissolve at higher temperature and which swells in aqueous media preventing water penetration inside to the core. The swelled stable gel further prevents the effect of the high temperature on the inner core. A number of polymers exhibit abrupt changes in their aqueous solubility with an increased temperature; the resulting sol-gel transition occurring at the lower critical solubility temperature (LCST) is characterized by minimal heat production and absence of byproducts. The "cloud point" represents the temperature at which a water-soluble compound begins to come out of solution with resulting scattering of light or "cloud" formation. The polymer-polymer and the polymer-solvent interactions (solvent that in food applications will be usually water) show an abrupt re-adjustment in small ranges of temperature, and this is translated to a chain transition between extended and compacted coil states. Temperature-responding polymers present a fine hydrophobic-hydrophilic balance in their structure, and small temperature changes around the critical solubility temperature (LCST) make the chains collapse or expand, while responding to the new adjustments of the hydrophobic and hydrophilic interactions between the polymeric chains and the aqueous media.

Considering the free energy of association ($\Delta G$) between the polymer chains:

$$\Delta G = \Delta H - T\Delta S$$

where $\Delta H$ is the enthalpy term, $\Delta S$ the entropy term and T temperature, it can be concluded that increase over a critical temperature results in a larger value of $T\Delta S$ than the positive enthalpy term ($\Delta H$), and thus a negative $\Delta G$ favoring polymer association: chain-chain interactions (hydrophobic effects, hydrogen bonding) dominate over chain-water hydrogen bonding. On the other hand upon decreasing temperature below a critical temperature, water hydrogen bonding dominates over chain-chain interactions thus the dissolution of the polymer may occur. Macroscopic response of the polymer will depend on the physical state of the chains. If the macromolecular chains are linear and solubilized, the solution will change from mono-phasic to biphasic due to polymer precipitation when the transition occurs. Polymer solution is a free-flowing liquid at ambient temperature and gels at high temperature. In some cases, if lowering the amount of thermo-gelling polymer is necessary, it may be blended with a pH-sensitive reversibly gelling polymer.

Block copolymers containing one block with a LCST at a temperature range where the other block is soluble, self assemble in response to temperature increase. Morphology of the self-assembled structure depends on copolymer architecture and MW; micelles or networks of infinite MW (gels) can be obtained by appropriate design. A recently reported, alternative approach was based on interpenetrating networks of poly(N-isopropylacrylamide) (PNIPAM) and poly(acrylic acid) (PAAc), formulated in nanoparticles. The collapse of PNIPAM above its LCST triggered the bonding of the NPs into a network while the repulsion between the charged PAAc chains prevented agglomeration.

The thermo-sensitive polymers exhibiting thermally-driven phase transitions is selected from the group consisting of poly-N-substituted acrylamide derivatives such as poly(N-isopropylacrylamide) (PNIPAM), Poly-N-acryloylpiperidine, Poly-N-propylmethacrylamide, Poly-N-isopropylacrylamide Poly-N-diethylacrylamide, Poly-N-isopropylmethacrylamide, Poly-N-cyclopropylacrylamide, Poly-N-acryloylpyrrolidine, Poly-N,N-ethylmethylacrylamide, Poly-N-cyclopropylmethacrylamide, Poly-N-ethylacrylamide, poly-N-substituted methacrylamide derivatives, copolymers comprising an N-substituted acrylamide derivative and an N-substituted methacrylamide derivative, copolymer of N-isopropylacrylamide and acrylic acid, polypropyleneoxide, polyvinylmethylether, partially-acetylated product of polyvinyl alcohol, copolymers comprising propyleneoxide and another alkylene oxide such as non-ionic, amphiphilic poly(ethylene glycol)-bl-poly(propylene glycol)-bl-poly(ethylene glycol) (PEGPPG-PEG) block copolymer (also referred to as Tetronics®, poloxamer, Pluronic®), Poloxamer-co-PAAc, Oligo(poloxamers), Methylcellulose (MC), hydroxylpropylcellulose (HPC), methylhydroxyethylcelluloce (MHEC), hydroxylpropylmethylcellulose (HPMC), hydroxypropylethylcellulose (HPEC), hydroxymethylpropylcellulose (HMPC), ethylhydroxyethylcellulose (EHEC) (Ethulose), hydroxyethylmethylcellulose (HEMC), hydroxymethylethylcellulose (HMEC), propylhydroxyethylcellulose (PHEC), hydrophobically modified hydroxyethylcellulose (NEXTON), amylose, amylopectin, Poly(organophosphazenes), natural polymers like xyloglucan, or a mixture thereof.

The above mentioned poly-N-substituted acrylamide derivatives may be either a homopolymer or a copolymer comprising a monomer constituting the above polymer and "another monomer". The "another monomer" to be used for such a purpose may be a hydrophilic monomer, or a hydrophobic monomer. In general, when copolymerization with a hydrophilic monomer is conducted, the resultant cloud point temperature may be increased. On the other hand, when copolymerization with a hydrophobic monomer is conducted, the resultant cloud point temperature may be decreased. Accordingly, a polymer having a desired cloud point (e.g., a cloud point of higher than 30° C), may be obtained by selecting monomers to be used for copolymerization.

Specific examples of the above hydrophilic monomers include: N-vinyl pyrrolidone, vinylpyridine, acrylamide, methacrylamide, N-methylacrylamide, hydroxyethylmethacrylate, hydroxyethylacrylate, hydroxymethylmethacrylate, hydroxymethylacrylate, methacrylic acid and acrylic acid having an acidic group, and salts of these acids, vinylsulfonic acid, styrenesulfonic acid, etc., and derivatives having a basic group such as N,N-dimethylaminoethylmethacrylate, N,N-diethylaminoethyl methacrylate, N,N-dimethylaminopropylacrylamide, salts of these derivatives, etc. However, the hydrophilic monomer to be usable in the present invention is not restricted to these specific examples.

On the other hand, specific examples of the above hydrophobic monomer may include acrylate derivatives and methacrylate derivatives such as ethylacrylate, methylmethacrylate, and glycidylmethacrylate; N-substituted alkymethacrylamide derivatives such as N-n-butylmethacrylamide; vinylchloride, acrylonitrile, styrene, vinyl acetate, etc. However, the hydrophobic monomer to be usable in the present invention is not restricted to these specific examples.

Among the polymers that show thermosensitive character is poly (ethylene oxide)-poly (propylene oxide)-poly (ethylene oxide) triblock copolymers (PEO-PPO-PEO) (Pluronics® or Poloxamers®) which is a family of ABA-type triblock copolymer consisting of more than 30 non-ionic amphiphilic copolymers (Figure 2). The physical state (liquid, paste, solid) of these copolymers is governed by their MW and block ratio. Poloxamers are well tolerated (non-toxic) biocompatible polymer. These block copolymers show gelation at body temperature at concentrations greater than 15% (w/w). The above-described property of the blocks having a cloud point is caused by hydrophobic bond of the blocks whose strength increases with an increase in temperature and decreases with a decrease in temperature. In the present invention hydrophobic bonds form between the cloud point blocks replacing the bonds between the blocks and the water molecules, thereby causing the blocks to become insoluble. The presence of hydrophilic blocks imparts the polymer with the ability to form a water-containing gel rather than being precipitated at a temperature higher than the cloud point temperature due to an excess increase in the hydrophobic bonding strength of the cloud point blocks. The coexistence of the cloud point blocks and the hydrophilic blocks in the polymer causes it to be converted from a water-soluble sol state below the temperature into a water-insoluble gel state at a temperature at or above the cloud point temperature, which temperature essentially corresponds to the sol-gel transition temperature of the polymer (Figure 3).

On the other hand, in the case of an etherified cellulose represented by methylcellulose, hydroxypropylcellulose, etc., the sol-gel transition temperature thereof is as high as about 45°C or higher. Hydroxypropylcellulose (HPC) is an example of a thermo-sensitive polymer. HPC is an ether of cellulose in which some of the hydroxyl groups in the repeating glucose units have been hydroxypropylated forming $-OCH_2CH(OH)CH_3$ groups using propylene oxide. The average number of substituted hydroxyl groups per glucose unit is referred to as the degree of substitution (DS). Complete substitution would provide a DS of 3. Because the hydroxypropyl group added contains a hydroxyl group, this can also be etherified during preparation of HPC. When this occurs, the number of moles of hydroxypropyl groups per glucose ring, moles of substitution (MS), can be higher than 3. Since hydroxypropyl cellulose (HPC) has a combination of hydrophobic and hydrophilic groups, so it also has a lower critical solution temperature (LCST) at 45 °C. At temperatures below the LCST, HPC is readily soluble in water; above the LCST, HPC is not soluble (Figure 4).

According to the invention, said outer heat resisting coating layer (first layer) comprises HPC LF or HPC EF and Poloxamer 124

The exterior coating layer: According to further features in any of the embodiments of the invention, the encapsulated probiotics optionally and preferably further comprises an outermost (exterior) coating layer which is preferably a water soluble polymer layer for enhancing the dissolution of said thermo-sensitive gel forming polymer on cooling below its cloud point or its lower critical solution temperature (LCST). Example of materials that may be used for the outermost coating layer is selected from the group consisting of water soluble or erodible polymers such as, for example, Povidone (PVP: polyvinyl pyrrolidone), Copovidone (copolymer of vinyl pyrrolidone and vinyl acetate), polyvinyl alcohol, Kollicoat Protect (BASF) which is a mixture of Kollicoat IR (a polyvinyl alcohol (PVA)-polyethylene glycol (PEG) graft copolymer) and polyvinyl alcohol (PVA), Opadry AMB (Colorcon) which is a mixture based on PVA, Aquarius MG which is a cellulose-based polymer containing natural wax, lecithin, xanthan gum and talc, low molecular weight HPC (hydroxypropyl cellulose), low molecular weight HPMC (hydroxypropyl methylcellulose) such as hydroxypropylcellulose (HPMC E3 or E5) (Colorcon), methyl cellulose (MC), low molecular weight carboxy methyl cellulose (CMC), low molecular weight carboxy methyl ethyl cellulose (CMEC), low molecular weight hydroxyethylcellulose (HEC), low molecular weight hydroxyl ethyl methyl cellulose (HEMC), low molecular weight hydroxymethylcellulose (HMC), low molecular weight hydroxymethyl hydroxyethylcellulose (HMHEC), low viscosity of ethyl cellulose, low molecular weight methyl ethyl cellulose (MEC), gelatin, hydrolyzed gelatin, polyethylene oxide, water soluble gums, water soluble polysaccharides, acacia, dextrin, starch, modified cellulose, water soluble polyacrylates, polyacrylic acid, polyhydroxyethylmethacrylate (PHEMA) and polymethacrylates and their copolymers, and/or a mixtures thereof.

According to the invention, said exterior coating layer (second layer) comprises HPMC E5 and polyethylene glycol.

Substrate: According to a preferred embodiment of the invention, the probiotic bacteria in said granule core are mixed with a substrate. Said substrate preferably comprises at least one material that may be also a supplement agent for the probiotic bacteria. The substrate may comprise monosaccharides such as trioses including ketotriose (dihydroxyacetone) and aldotriose (glyceraldehyde), tetroses such as ketotetrose (erythrulose), aldotetroses (erythrose, threose) and ketopentose (ribulose, xylulose), pentoses such as aldopentose (ribose, arabinose, xylose, lyxose), deoxy sugar (deoxyribose) and ketohexose (psicose, fructose, sorbose, tagatose), hexoses such as aldohexose (allose, altrose, glucose, mannose, gulose, idose, galactose, talose), deoxy sugar (fucose, fuculose, rhamnose) and heptose such as (sedoheptulose), and octose and nonose (neuraminic acid). The substrate may comprise multiple saccharides such as 1) disaccharides, such as sucrose, lactose, maltose, trehalose, turanose, and cellobiose, 2) trisaccharides such as raffinose, melezitose and maltotriose, 3) tetrasaccharides such as acarbose and stachyose, 4) other oligosaccharides such as fructooligosaccharide (FOS), galactooligosaccharides (GOS) and mannan-oligosaccharides (MOS), 5) polysaccharides such as glucose-based polysaccharides/glucan including glycogen starch (amylose, amylopectin), cellulose, dextrin, dextran, beta-glucan (zymosan, lentinan, sizofiran), and maltodextrin, fructose-based polysaccharides/fructan including inulin, levan beta 2-6, mannose-based polysaccharides (mannan), galactose-based polysaccharides (galactan), and N-acetylglucosamine-based polysaccharides including chitin. Other polysaccharides may be comprised, including gums such as arabic gum (gum acacia).

According to preferred embodiments of the present invention, the core further comprises an antioxidant. Preferably, the antioxidant is selected from the group consisting of cysteine hydrochloride, cystein base, 4,4-(2,3 dimethyl tetramethylene dipyrocatechol), tocopherol-rich extract (natural vitamin E), α-tocopherol (synthetic Vitamin E), β-tocopherol, γ-tocopherol, δ-tocopherol, butylhydroxinon, butyl hydroxyanisole (BHA), butyl hydroxytoluene (BHT), propyl gallate, octyl gallate, dodecyl gallate, tertiary butylhydroquinone (TBHQ), fumaric acid, malic acid, ascorbic acid (Vitamin C), sodium ascorbate, calcium ascorbate, potassium ascorbate, ascorbyl palmitate, and ascorbyl stearate. Comprised in the core may be citric acid, sodium lactate, potassium lactate, calcium lactate, magnesium lactate, anoxomer, erythorbic acid, sodium erythorbate, erythorbin acid, sodium erythorbin, ethoxyquin, glycine, gum guaiac, sodium citrates (monosodium citrate, disodium citrate, trisodium citrate), potassium citrates (monopotassium citrate, tripotassium citrate), lecithin, polyphosphate, tartaric acid, sodium tartrates (monosodium tartrate, disodium tartrate), potassium tartrates (monopotassium tartrate, dipotassium tartrate), sodium potassium tartrate, phosphoric acid, sodium phosphates (monosodium phosphate, disodium phosphate, trisodium phosphate), potassium phosphates (monopotassium phosphate, dipotassium phosphate, tripotassium phosphate), calcium disodium ethylene diamine tetra-acetate (calcium disodium EDTA), lactic acid, trihydroxy butyrophenone and thiodipropionic acid and mixtures thereof. According to one preferred embodiment, the antioxidant is cystein base.

According to some embodiments of the present invention, the core further comprises both filler and binder. Examples of fillers include, for example, microcrystalline cellulose, a sugar, such as lactose, glucose, galactose, fructose, or sucrose; dicalcium phosphate; sugar alcohols such as sorbitol, manitol, mantitol, lactitol, xylitol, isomalt, erythritol, and hydrogenated starch hydrolysates; corn starch; potato starch; sodium carboxymethycellulose, ethylcellulose and cellulose acetate, or a mixture thereof. More preferably, the filler is lactose. Examples of binders include Povidone (PVP: polyvinyl pyrrolidone), Copovidone (copolymer of vinyl pyrrolidone and vinyl acetate), polyvinyl alcohol, low molecular weight HPC (hydroxypropyl cellulose), low molecular weight HPMC (hydroxypropyl methylcellulose), low molecular weight carboxy methyl cellulose, low molecular weight hydroxyethylcellulose, low molecular weight hydroxymethylcellulose, gelatin, hydrolyzed gelatin, polyethylene oxide, acacia, dextrin, starch, and water soluble polyacrylates and polymethacrylates, low molecular weight ethylcellulose or a mixture thereof.

Examples of probiotic bacteria include but are not limited to *Bacillus coagulans GBI-30, 6086, Bacillus subtilis var natt, Bifidobacterium LAFTI® B94, Bifidobacterium sp LAFTI B94, Bifidobacterium bifidum, Bifidobacterium bifidum rosell-71, Bifidobacterium breve, Bifidobacterium breve Rosell-70, Bifidobacterium infantis, Bifidobacterium lactis, Bifidobacterium longum, Bifidobacterium longum Rosell-175, Bifidobacterium animalis, Bifidobacterium animalis subsp. lactis BB-12, Bifidobacterium animalis subsp. lactis HN019, Bifidobacterium infantis 35624, Escherichia coli M-17, Escherichia coli Nissle 1917, Lactobacillus acidophilus, Lactobacillus acidophilus LAFTI® L10, Lactobacillus acidophilus LAFTI L10, Lactobacillus casei LAFTI® L26, Lactobacillus casei LAFTI L26, Lactobacillus brevis, Lactobacillus bulgaricus, Lactobacillus casei, Lactobacillus gasseri, Lactobacillus paracasei, Lactobacillus plantarum, Lactobacillus reuteri ATTC 55730 (Lactobacillus reuteri SD2112), Lactobacillus rhamnosus, Lactobacillus salivarius, Lactobacillus delbrueckii, Lactobacillus fermentum, Lactococcus lactis, Lactococcus lactis subsp, Lactococcus lactis Rosell-1058, Lactobacillus paracasei St11 (or NCC2461) Lactobacillus fortis Nestle, Lactobacillus johnsonii La1 (= Lactobacillus LC1, Lactobacillus johnsonii NCC533) Nestlé, Lactobacillus rhamnosus Rosell-11, Lactobacillus acidophilus Rosell-52, Streptococcus thermophilus, Diacetylactis, or other microorganisms like Saccharomyces cerevisiae,* and a mixture thereof.

The invention enables to manufacture various healthy food products without separating the admixing heating steps. Enabled is, for example, the preparation of liquid-based products containing the probiotic granules, avoiding any awkward injecting steps of prior art methods. The encapsulated pro-biotic bacteria according to the present invention may be incorporated into infant foods such as infant food powder compound, into liquid-based products mainly those that undergo heating steps during their manufacturing and those whose final transparency and appearance are an important marketing factor, into hot drinks, into nectars and into fruit juices, and into other beverage products that may be exposed to higher than ambient temperature (room temperature) during their handling and/or production.

The invention will be further described and illustrated in the following examples.

## Examples

### Example 1

Materials

| Materials: | Function: |
|---|---|
| *Bifidobacterium lactis* | A Probiotic bacteria |
| Maltodextrin | Core substrate |
| Trehalose | Core substrate |
| HPMC E3 | Core Binder |
| HPMC E5 | First coating layer agent |
| PEG 2000 | Plasticizer |
| HPC LF | Second coating layer polymer |
| Poloxamer 124 | Second coating layer polymer |

Method

*Bifidobacterium lactis* (BL 818)(44.8 g), maltodextrin (402.3 g) and trehalose (51.1 g) were granulated with a solution of hydroxypropylmethyl cellulose (HPMC E3) (50.8 g) in water (7-10% W/W) using an Innojet Ventilus coater machine. The resulting granules (518.9 g) were then coated by a sub-coating layer comprising hydroxypropylmethyl cellulose (HPMC E5) (88.1 g) and polyethylene glycol (PEG 2000) (17.6 g) using a 7% (W/W) solution in water to obtain 20% (W/W) weight gain. 500 g of the resulting coated granules were then coated with an outer coating comprising hydroxypropyl cellulose (HPC LF) (88.1 g) and Poloxamer 124 (17.6 g) using a 5% (W/W) solution in water. Samples were taken for heat resistance test in liquid media, scanning electron microscopy (SEM) and particle size distribution analysis after 20%, 40%, 50%, 60% and 70% (W/W) weight gain of the outer coating.

**Example 2**

Materials

| Materials: | Function: |
|---|---|
| *Bifidobacterium lactis* | A Probiotic bacteria |
| Maltodextrin | Core substrate |
| Trehalose | Core substrate |
| HPMC E3 | Core binder |
| HPMC E5 | First coating layer agent |
| PEG 2000 | Plasticizer |
| HPC EF | Second coating layer polymer |
| Poloxamer 124 | Second coating layer polymer |

Method

*Bifidobacterium lactis* (BL 818)(44.8 g), maltodextrin (402.3 g) and trehalose (51.1 g) were granulated with a solution of hydroxypropylmethyl cellulose (HPMC E3) (50.8 g) in water (7-10% W/W) using an Innojet Ventilus coater machine. The resulting granules (518.9 g) were then coated by a sub-coating layer comprising hydroxypropylmethyl cellulose (HPMC E5) (88.1 g) and polyethylene glycol (PEG 2000) (17.6 g) using a 7% (W/W) solution in water to obtain 20% (W/W) weight gain. 500 g of the resulting coated granules were then coated with an outer coating comprising hydroxypropyl cellulose (HPC EF) (88.1 g) and Poloxamer 124 (17.6 g) using a 7% (W/W) solution in water. Samples were taken for heat resistance test in liquid media after 20%, 50% and 70% (W/W) weight gain of the outer coating.

**Example 3**

Materials

| Materials: | Function: |
|---|---|
| *Bifidobacterium lactis* | A Probiotic bacteria |
| Maltodextrin | Core substrate |
| Trehalose | Core substrate |
| HPMC E3 | Core Binder |
| HPMC E5 | Second coating layer agent |
| PEG 2000 | Plasticizer |

(continued)

| Materials: | Function: |
|---|---|
| HPC EF | First coating layer polymer |
| Poloxamer 124 | First coating layer polymer |

Method

*Bifidobacterium lactis* (BL 818)(45 g), maltodextrin (405 g) and trehalose (52 g) were granulated with a solution of hydroxypropylmethyl cellulose (HPMC E3) (45.2 g) in water (10% W/W) using an Innojet Ventilus coater machine. The resulting granules (500 g) were then coated by a sol-gel coating layer comprising hydroxypropyl cellulose (HPC EF) (204.1 g) and Poloxamer 124 (40.8 g) using a 7.8% (W/W) solution in water to obtain a weight gain of 33.6% (W/W). The resulting granules (500 g) were then coated with an outer coating layer comprising hydroxypropylmethyl cellulose (HPMC E5) (88.1 g) and polyethylene glycol (PEG 2000) (17.6 g) using a 7.4% (W/W) solution in water to obtain 20% (W/W) weight gain. The sample was then tested for heat resistance in both NaCl solution as well as infant milk formulation suspension.

HEAT RESISTANCE TEST METHOD IN SOLUTION OF NACL (0.9%) IN PURIFIED WATER

Objective

Evaluation of the survival rate of microencapsulated bacteria according to the present invention. The test was done by dispersing the sample of microencapsulated bacteria particles in preheated NaCl solution (0.9%) in purified water at 70°C for 5 minutes.

Principle of the method

1. Sample of microencapsulated Probiotic particles are dispersed in water (NaCl solution, 0.9%) which preheated to 70°C.
2. After 5 minutes the water (NaCl solution 0.9%) is cooled down to below 40°C.
3. Microencapsulated probiotic particles is completely dissolved.
4. Enumeration test is performed to determine the colony forming units per gram of the bacteria content in the sample (CFU/g).
5. The results will be compared to those of blank samples (the bacteria without microencapsulation).
6. Control samples will be prepared by dissolution of both microencapsulated bacteria and the bacteria without microencapsulation directly in water (NaCl solution 0/9%) at room temperature (with no preheating).

Procedure for heat resistance test method

1. Weigh accurately 10 gram of the probiotic sample (either microencapsulated bacteria particles according to the present invention or the bacteria without microencapsulation).
2. Put 100 ml distilled water (NaCl solution 0.9%) in a glass beaker and heat to 70°C using a bath equipped with a thermostat.
2. Measure and note the temperature.
3. Introduce the weighed sample into the water (NaCl solution 0.9%) and immediately start measuring time.
4. After 5 minutes accurately take out the glass beaker from the bath and cool down to 40°C.
5. Dissolve completely the sample of the microencapsulated bacteria particles using a shaker for about 0.25-4 hours depending on the weight gain of thermo-sensitive gel-forming coating layer.
6. Perform enumeration test and calculate CFU/gr.

Procedure for control sample

1. Weigh accurately 10 gram of the probiotic sample (either microencapsulated bacteria particles according to the present invention or the bacteria without microencapsulation).
2. Disperse the weighed sample into 100 ml water (NaCl solution 0.9%) at room temperature.
3. Dissolve completely the sample of the microencapsulated bacteria particles using a shaker for about 0.25-4 hours depending on the weight gain of thermo-sensitive gel-forming coating layer.
4. Perform enumeration test and calculate CFU/gr.

HEAT RESISTANCE TEST METHOD IN INFANT MILK

FORMULATION SUSPENSION

Objective

Evaluation of the survival rate of microencapsulated bacteria according to the present invention. The test was done by dispersing the sample of microencapsulated bacteria particles in infant milk formulation suspension at 70°C for 5 minutes.

Principle of the method

1. Sample of microencapsulated Probiotic particles are dispersed in particles in infant milk formulation suspension at 70°C for 5 minutes.
2. After 5 minutes infant milk formulation suspension is cooled down to below 40°C.
3. The infant milk formulation suspension is shaken to dissolve microencapsulated probiotic particles.
4. Enumeration test is performed to determine the colony forming units per gram of the bacteria content in the sample (CFU/g).

Procedure for control sample

1. Heat 210 ml of water to 70°C and put into the flask.
2. Disperse mix powder of sample and infant milk powder into the flask.
3. Close the flask, turn vertical, shake 30x up and down.
4. Cool down milk, place the flask at room temperature until milk temperature is 37°C (slow cooling); time estimation: 30 min.
5. Perform enumeration test and calculate CFU/gr.

Preparation of mix powder of sample and infant milk powder

Mix powder I. 3.2 g of sample and 28.8 g of infant milk powder,
mix powder II. 9.6 g of sample and 22.4 g of infant milk powder,
mix powder III. 16 g of sample and 16 g of infant milk powder.

RESULTS

1. Heat resistance test results in NaCl solution (0.9%)

1.1. Heat resistance test results in NaCl solution (0.9%), with a regular dissolution time (0.25 h), of microencapsulated probiotic bacteria containing different weight gains of thermo-sensitive sol-gel coating layer according to the present invention.

Example 1 as compared to uncoated bacteria mixed with maltodextrin with the ratio of 1:9 (Maltodextrin: BL818 mixture) respectively at both room temperature and 70°C.

| Sample | Test at room temperature | Test at 70°C |
|---|---|---|
| Maltodextrin: BL818 mixture (9:1) | 2.8 x 10 exp 9 | 0 |
| Example 1, 20% weight gain * | 6.1 x 10 exp 8 | 0 |
| Example 1, 40% weight gain* | 6.0 x 10 exp 8 | 0 |
| Example 1, 50% weight gain* | 4.7 x 10 exp 8 | 8.3 x 10 exp 7 |
| Example 1, 70% weight gain* | 0 | 0 |
| * The percentage of weight gain indicates the weight gain resulted from the coating by the thermo-sensitive sol-gel coating layer | | |

1.2 Heat resistance test results in NaCl solution (0.9%) with an extended dissolution time (2 hours and 4 hours) of microencapsulated probiotic bacteria containing different weight gains of thermo-sensitive sol-gel coating layer according to the present invention, Example 1, as compared to uncoated bacteria mixed with maltodextrin with the ratio of 1:9 (Maltodextrin: BL818 mixture) respectively.

| Sample | Test at room temperature | Test at 70°C |
|---|---|---|
| Example 1, 50% weight gain Extended dissolution time (2 hours)* | 1.5 x 10 exp 9 | 1.6 x 10 exp 9 |
| Example 1, 60% weight gain Extended dissolution time (2 hours)* | 1.2 x 10 exp 9 | 3.6 x 10 exp 6 |
| Example 1, 70% weight gain Extended dissolution time (2 hours)* | 1.8 x 10 exp 7 | 9.3 x 10 exp 5 |
| Example 1, 70% weight gain Extended dissolution time (4 hours)* | 8.2 x 10 exp 8 | 8.8 x 10 exp 8 |
| Example 1, 70% weight gain Extended dissolution time (4 hours)- retest* | 1.0 x 10 exp 8 | 2.1 x 10 exp 8 |
| * The percentage of weight gain indicates the weight gain resulted from the coating by the thermo-sensitive sol-gel coating layer | | |

1.3 Heat resistance test results in NaCl solution (0.9%), with a regular dissolution time (0.25 h), of microencapsulated probiotic bacteria according to the present invention, Example 3, as compared to uncoated bacteria mixed with maltodextrin with the ratio of 1:9 (Maltodextrin: BL818 mixture) respectively at both room temperature and 70°C.

| Sample | Test at room temperature | Test at 70°C |
|---|---|---|
| Maltodextrin: BL818 mixture (9:1) | 2.1 x 10 exp 9 | 0 |
| Example 3 | 2.2 x 10 exp 9 | 2.2 x 10 exp 9 |

2. Heat resistance test method in infant milk formulation suspension

Heat resistance test results of microencapsulated probiotic bacteria according to the present invention, Example 3, in infant milk suspension at 70°C using different mix powders of sample and infant milk powder.

| Sample | Mix powder of sample and infant milk powder | Test at 70°C |
|---|---|---|
| Example 3 | mix powder I | 3.2 x 10 exp 9 |
| Example 3 | mix powder II | 3.7 x 10 exp 9 |
| Example 3 | mix powder III | 3.2 x 10 exp 9 |

Scanning Electron Microscopy (SEM) Analysis

In order to investigate the structure of the surface of microencapsulated probiotics, SEM analysis of the samples prepared according to Example 1 was performed. Generally the surface of the microencapsulated bacteria samples should not be porous. Porosity may cause capillary effect which may eventually increase the penetration of hot water into the core and thus enhance the destruction of the probiotics at elevated temperatures. The surface of the film coat should be sealed and adequately tight in order to block as much as possible the penetration of water at elevated temperature (for example 70°C) into the core prior to the formation gel by the thermo-sensitive sol-gel coating layer.

Particle size and particle size distribution analysis

Particle size distribution of microencapsulated *Bifidobacterium lactis* (BL818) samples prepared according to Example 1 and Example 2 of the present invention was measured in water using a Malvern Mastersizer. 10 g of sample was placed in a glass vessel and 90 ml of preheated water (70°C) was added and stirred to ensure total dispersion of the sample. After 5 minutes the temperature was cooled down for 30 minutes during which the dispersion was stirred every 5-10 minutes manually using a glass rod. The sample was then taken to the Mastersizer for particle size measurement. The sample was stirred mechanically at Mastersizer. The integral and differential curves revealing the particle size distribution of the samples are shown in Figures 5-7.

**Claims**

**1.** Stabilized probiotic granules for admixing to a liquid-based food product, resistant to heating in an aqueous environment, comprising

i) a core of probiotic bacteria in a substrate or mixed with a substrate;
ii) at least one first layer adjacent to the core which is an outer layer comprising a thermo-reversible gel-forming polymer having a lower critical solution temperature (LCST) of at least 45 °C selected from HPC LF or HPC EF in combination with Poloxamer 124,
iii) an inner layer, which is layered between said core of probiotic bacteria and said first layer (outer layer) comprising the thermo-reversible gel forming polymer, wherein said inner layer is selected from the group consisting of water soluble or erodible polymers, wherein said inner layer comprises HPMC E5 and polyethylene glycol;
iv) optionally a second layer adjacent to said outer layer, which is an exterior layer comprising a water soluble or erodible polymer for enhancing the dissolution of said thermo-reversible gel forming polymer on cooling below its lower critical solution temperature (LCST), wherein said second layer comprises HPMC E5 and polyethylene glycol.

**2.** Stabilized probiotic granules according to claim 1, wherein said food product is selected from the group consisting of infant food products and powdered infant formula adapted to be suspended in hot water at about 70°C.

**3.** Stabilized probiotic granules according to claim 1 or 2, wherein said substrate comprises a component selected from the group consisting of supplement for bacteria, prebiotic saccharide, stabilizer, filler, binder, and a mixture thereof.

**4.** An infant powdered food comprising the granules of claim 1.

**Patentansprüche**

**1.** Stabilisiertes probiotisches Granulat zum Zumischen zu einem Nahrungsmittelprodukt auf flüssiger Basis, das gegen Erhitzen in einer wässrigen Umgebung beständig ist, umfassend

i) einen Kern aus probiotischen Bakterien in einem Substrat oder gemischt mit einem Substrat;
ii) mindestens eine erste Schicht benachbart zu dem Kern, bei der es sich um eine äußere Schicht handelt, die ein thermoreversibles gelbildendes Polymer mit einer niedrigeren kritischen Lösungstemperatur (LCST) von mindestens 45 °C umfasst, ausgewählt aus HPC LF oder HPC EF in Kombination mit Poloxamer 124,
iii) eine innere Schicht, die zwischen dem Kern aus probiotischen Bakterien und der ersten Schicht (äußeren Schicht), die das thermoreversible gelbildende Polymer umfasst, geschichtet ist, wobei die innere Schicht aus der Gruppe ausgewählt ist, die aus wasserlöslichen oder erodierbaren Polymeren besteht, wobei die innere Schicht HPMC E5 und Polyethylenglykol umfasst;

iv) optional eine zweite Schicht benachbart zu der äußeren Schicht, die eine externe Schicht ist, die ein wasserlösliches oder erodierbares Polymer umfasst, um die Auflösung des thermoreversiblen gelbildenden Polymers beim Abkühlen unter dessen niedrigere kritische Lösungstemperatur (LCST) zu verbessern, wobei die zweite Schicht HPMC E5 und Polyethylenglykol umfasst.

2. Stabilisiertes probiotisches Granulat nach Anspruch 1, wobei das Nahrungsmittelprodukt ausgewählt ist aus der Gruppe, bestehend aus Säuglingsnahrungsprodukten und pulverförmiger Säuglingsnahrung, die in heißem Wasser bei etwa 70 °C suspendiert werden kann.

3. Stabilisiertes probiotisches Granulat nach Anspruch 1 oder 2, wobei das Substrat eine Komponente umfasst, die aus der Gruppe ausgewählt ist, die aus einem Zusatz für Bakterien, einem präbiotischen Saccharid, einem Stabilisator, einem Füllstoff, einem Bindemittel und einer Mischung davon besteht.

4. Pulverförmiges Säuglingsnahrungsmittel, umfassend das Granulat nach Anspruch 1.

**Revendications**

1. Granulés probiotiques stabilisés destinés à être mélangés à un produit alimentaire à base liquide, résistant au chauffage dans un environnement aqueux, comprenant

i) un noyau de bactéries probiotiques dans un substrat ou mélangé avec un substrat ;

ii) au moins une première couche adjacente au noyau qui est une couche externe comprenant un polymère gélifiant thermoréversible ayant une température de solution critique inférieure (LCST) d'au moins 45 C choisi parmi HPC LF ou HPC EF en combinaison avec le Poloxamer 124,

iii) une couche interne, qui est stratifiée entre ledit noyau de bactéries probiotiques et ladite première couche (couche externe) comprenant le polymère gélifiant thermoréversible, ladite couche interne étant choisie dans le groupe constitué des polymères solubles ou érodables dans l'eau, ladite couche interne comprenant du HPMC E5 et du polyéthylène glycol ;

iv) éventuellement une seconde couche adjacente à ladite couche externe, qui est une couche extérieure comprenant un polymère soluble ou érodable dans l'eau pour améliorer la dissolution dudit polymère gélifiant thermoréversible lors du refroidissement en dessous de sa température de solution critique inférieure (LCST), ladite seconde couche comprenant du HPMC E5 et du polyéthylène glycol.

2. Granulés probiotiques stabilisés selon la revendication 1, dans lesquels ledit produit alimentaire est choisi dans le groupe constitué des produits alimentaires pour nourrisson et des préparations en poudre pour nourrisson adaptés pour être suspendus dans de l'eau chaude à environ 70 C.

3. Granulés probiotiques stabilisés selon la revendication 1 ou 2, dans lesquels ledit substrat comprend un composant choisi dans le groupe constitué d'un supplément pour bactéries, d'un saccharide prébiotique, d'un stabilisant, d'une charge, d'un liant et d'un mélange de ceux-ci.

4. Aliment en poudre pour nourrisson comprenant les granulés de la revendication 1.

Fig. 1

$$HO-(CH_2\text{-}CH_2\text{-}O)_n-(\overset{\overset{\textstyle CH_3}{|}}{C}H_2\text{-}CH_2\text{-}O)_m-(CH_2\text{-}CH_2\text{-}O)_n-H$$

## Fig. 2A

Fig. 2B

Fig. 3A

PO block

EO block

T increase

micellization

T increase

gelation

Fig. 3B

Fig. 4A

Fig. 4B

**Fig. 5**

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050019417 A1
- US 20100055083 A
- US 20100074994 A
- US 20051053018 A
- US 6290988 B